# FASCICULE DE BREVET EUROPEEN

(11) **EP 1 480 687 B1**
(45) Date de publication et mention de la délivrance du brevet: **11.02.2009**
(21) Numéro de dépôt: 03717407.5
(22) Date de dépôt: 18.02.2003
(51) Int. Cl.: A61L 27/12, A61L 27/54

(54) **COMPOSE PHOSPHOCALCIQUE MODIFIE, COMPOSITION INJECTABLE LE CONTENANT**
MODIFIZIERTE CALCIUMPHOSPHAT-VERBINDUNG UND INJIZIERBARE ZUSAMMENSETZUNG ENTHALTEND DIESELBE
MODIFIED PHOSPHOCALCIC COMPOUND, INJECTABLE COMPOSITION CONTAINING SAME

(30) Priorité: 04.03.2002 FR 0202707
(43) Date de publication de la demande: 01.12.2004
(73) Titulaire: CENTRE NATIONAL DE LA RECHERCHE SCIENTIFIQUE, 75016 Paris (FR); UNIVERSITE DE NANTES, 44000 Nantes (FR); INSTITUT NATIONAL DE LA SANTE ET DE LA RECHERCHE MEDICALE (INSERM), 75013 Paris (FR)
(72) Inventeur: BUJOLI, Bruno, F-44240 SUCE SUR ERDRE (FR); JOSSE, Solen, F-44360 CORDEMAIS (FR); GUICHEUX, Jérôme, F-44115 HAUTE GOULAINE (FR); JANVIER, Pascal, F-44000 NANTES (FR); BOULER, Jean-Michel, F-44300 NANTES (FR); DACULSI, Guy, F-44360 VIGNEUX DE BRETAGNE (FR)
(74) Mandataire: Cordier, Pascal Christian
(86) Numéro de dépôt international: PCT/FR2003/000527
(87) Numéro de publication internationale: WO 2003/074098

(56) Documents cités:
- WO-A-00/47214
- WO-A-95/21634
- DENISSEN H ET AL: "CERAMIC HYDROXYAPATITE IMPLANTS FOR THE RELEASE OF BIPHOSPHONATE" BONE AND MINERAL, SHANNON, IE, vol. 25, no. 2, 1994, pages 123-134, XP001015353
- DENISSEN H ET AL: "Alveolar bone response to submerged bisphosphonate-complexed hydroxyapatite implants." JOURNAL OF PERIODONTOLOGY. UNITED STATES FEB 2000, vol. 71, no. 2, février 2000 (2000-02), pages 279-286, XP008010275 ISSN: 0022-3492 cité dans la demande
- DENISSEN H ET AL: "Net-shaped hydroxyapatite implants for release of agents modulating periodontal-like tissues." JOURNAL OF PERIODONTAL RESEARCH. DENMARK JAN 1997, vol. 32, no. 1 Pt 1, janvier 1997 (1997-01), pages 40-46, XP008010473 ISSN: 0022-3484

## Description

L'invention concerne un composé phosphocalcique modifié par un composé gem-biphosphonique, un procédé pour sa préparation, ainsi que son utilisation pour l'élaboration d'une composition injectable.

Le dérèglement de l'activité osseuse d'un individu est à l'origine de nombreuses pathologies osseuses telles que l'ostéoporose, la maladie de Paget ou les tumeurs ostéolytiques. Compte tenu notamment de l'augmentation de la longévité des personnes, l'ostéoporose est devenu un problème de santé publique et de nombreuses recherches ont été entreprises pour y remédier. Les pathologies osseuses considérées étant provoquées par un déséquilibre du remodelage osseux au profit de l'activité des ostéoclastes, l'une des voies de traitement envisagée a consisté à diminuer l'activité des ostéoclastes, en vue de ralentir la dégradation de la matière osseuse.

Les recherches effectuées sur divers acides gem-biphosphoniques ont montré leur pouvoir inhibiteur de l'activité des ostéoclastes (G. A. Rodan, et al., Therapeutic Approaches to Bone Diseases, 1 septembre 2000, vol. 289, Science, p.1508-1514). L'usage de certains d'entre eux comme médicament a été admis dans divers pays, notamment l'étidronate, le clodronate, le pamidronate, l'alendronate, le risedronate, le tiludronate et l'ibandronate. Des données ont été publiées pour d'autres composés acides gem-biphosphoniques, notamment le zoledronate, l'incadronate, l'olpadronate, le neridronate. Les acides gem-biphosphoniques qui sont utilisés à ce jour pour le traitement de lésions osseuses sont utilisés par voie systémique et génèrent de ce fait quelques effets secondaires indésirables. Ils peuvent provoquer des troubles rénaux lorsqu'ils sont administrés par voie intraveineuse, et des troubles du système digestif, notamment des oesophagites ou des ulcères de l'estomac, lorsqu'ils sont administrés par voie orale [(Lin J.H., Bone 1996 : 18 ; 75-85) ou (Thiébauld D. et al., Osteroporos Int 1994 ; 76-73)]. Un autre inconvénient de l'administration par voie orale réside dans le faible taux d'absorption du principe actif sur la matière osseuse.

On connaît par ailleurs des compositions injectables destinées à former des substituts osseux. FR-2715853 décrit des compositions pour bio-matériaux de résorption / substitution de tissus de soutien, comprenant une phase minérale composée de BCP ou de calcium-titane-phosphate, et une phase aqueuse liquide comprenant une solution aqueuse d'un polymère dérivé de la cellulose. Ces compositions injectables ne contiennent aucun principe actif.

Le document WO 00/47214 décrit une composition utilisable par injection pour le traitement d'ostéoporose ou de récidives de tumeurs lytiques par inhibition de l'activité ostéoclastique obtenue par mise en contact d'une pâte de ciment osseux avec un agent anti-résorptif tel qu'un bisphosphonate.

On connaît également des substituts osseux non injectables, qui se présentent sous forme d'implants. Par exemple, H. Denissen, et al (J. Periodontal, vol. 71, n°2, Février 2000, p. 280-296) décrit des implants d'hydroxyapatite modifiée par adsorption d'un acide gem-biphosphonique particulier, à savoir l'acide (3-diméthylamino-1-hydroxypropylidène)-1,1-biphosphonique ou olpadronate. La libération in situ de l'acide favoriserait la reconstruction osseuse. Cependant, l'hydroxyapatite elle-même présente l'inconvénient d'être très faiblement résorbable.

Le but de la présente invention est de fournir une composition contenant un principe actif inhibant l'activité des ostéoclastes et qui puisse être administrée sans produire les effets secondaires liés à une administration par voie systémique ou à l'utilisation d'un implant solide.

C'est pourquoi la présente invention a pour objet un composé phosphocalcique modifié, un procédé pour sa préparation, ainsi que son utilisation comme principe actif dans une composition injectable.

Le composé phosphocalcique modifié selon la présente invention peut être obtenu en ajoutant un acide gem-biphosphonique ou l'un de ses sels de métal alcalin ou alcalino-terreux à une suspension d'un composé phosphocalcique précurseur dans l'eau ultra-pure, en agitant le milieu réactionnel à température ambiante, puis en récupérant par centrifugation le composé formé. Le composé peut être ensuite purifié par lavage à l'eau ultra-pure, suivi d'une filtration et d'un séchage à l'air à température ambiante. Le composé phosphocalcique précurseur est choisi parmi les orthophosphates de calcium ayant une solubilité dans l'eau supérieure à 4.10⁻⁵⁹ mol.l⁻¹. A titre d'exemple, on peut citer les BCP qui sont un mélange d'hydroxyapatite et de phosphate tricalcique β (généralement désigné par β-TCP) en proportions variables, le CDA qui est une hydroxyapatite déficiente en calcium (obtenue par exemple par hydrolyse alcaline d'un orthophosphate de calcium acide), et le β-TCP.

Par eau ultra-pure, on entend dans ce texte une eau ayant une résistivité voisine de 18 MΩ cm.

L'agitation à température ambiante est maintenue de préférence pendant une durée entre 1 h et 72 h, par exemple pendant 48 heures. La nature de l'agitation et la granulométrie du composé phosphocalcique précurseur ont un effet sur la proportion de composé gem-biphosphonique qui peut être greffé. Il est donc préférable, lorsqu'une granulométrie donnée a été choisie pour le composé phosphocalcique précurseur, d'adapter l'agitation de sorte à ne pas modifier ladite granulométrie.

Les acides ou les sels qui peuvent être utilisés comme composés gem-biphosphoniques répondent à la formule (OY) (OX)P(O)-CR¹R² P(O) (OX) (OY) dans laquelle X ou Y représentent indépendamment l'un de l'autre H ou un cation de métal alcalin ou alcalino-terreux, R¹ représente H, OH ou un halogène, et R² représente :
- un hydrogène ou un halogène,
- un radical alkyle,
- un radical aminoalkyle dans lequel le groupe amino porte éventuellement un substituant alkyle,
- un radical alkylamino,
- un radical alkyle portant un substituant aromatique comprenant au moins un atome N,
- un radical alkyle portant un groupe thioéther aromatique. Lorsque R¹ et/ou R² représentent un halogène, Cl est particulièrement préféré.

Lorsque R² est un radical alkyle, on préfère les alkyles ayant de 1 à 6 atomes de carbone.

Lorsque R² est un radical aminoalkyle, on préfère les radicaux NH₂(CH)ₙ- dans lesquels n est inférieur à 6.

Lorsque R² est un radical alkylaminoalkyle, on préfère les radicaux R'R"N(CH₂)ₘ- dans lesquels R' et R" représentent indépendamment l'un de l'autre H, ou un radical alkyle ayant au plus 5 atomes de carbone, et m est inférieur à 6.

Lorsque R² est un radical alkylamino, on préfère les radicaux R^{c}NH- dans lesquels R^{c} est un cycloalkyle ayant de 3 à 7 atomes de carbone.

Lorsque R² est un radical alkyle portant un substituant aromatique comprenant au moins un atome N, on préfère les alkyles ayant au plus 3 atomes de carbone et portant un groupe pyridinyle ou imidazoyle.

Lorsque R² est un radical alkyle portant un groupe thioéther aromatique, on préfère les alkyles ayant au plus 3 atomes de carbone et portant un groupe phénylthio dans lequel le groupe phényle porte éventuellement un substituant halogène.

Parmi ces composés gem-biphosphoniques, on peut citer :
- l'étidronate (R¹ = OH, R² = CH₃) ,
- le clodronate (R¹ = Cl, R² = Cl),
- le pamidronate (R¹ = OH, R² = -CH₂CH₂NH₂),
- l'alendronate (R¹ = OH, R² = - (CH₂)₃NH₂),
- le risedronate (R¹ = OH, R² = -CH₂-3-pyridine),
- le tiludronate (R¹ = H, R² = -CH₂-S-C₆H₄-Cl),
- l'ibandronate (R¹ = OH, R² = -CH₂-CH₂-N(CH₃)pentyle),
- le zoledronate (R¹ = OH, R² = -CH₂-imidazole),
- l'incadronate (R¹ = H, R² = -NH-(cycloheptyle)),
- l'olpadronate (R¹ = OH, R² = CH₂-CH₂-N(CH₃)₂),
- le neridronate (R¹ = OH, R² = -(CH₂)₅NH₂).

Les acides dans lesquels R² est un radical alkyle portant un substituant aromatique comprenant au moins un atome N, tels que le zoledronate ou le risedronate, sont particulièrement préférés.

Un composé phosphocalcique modifié selon l'invention est caractérisé par la composition chimique suivante :

Ca₍₁₀₋ₐ) (Mg,K,Na)_{b}(PO₄)_{6-c}(HPO₄,CO₃)_{d}(OH)₂₋ₑ(F,Cl,CO₃)_{f}

[(OA) (OE) P (O) -CR¹R²-P (O) (OA) (OE)]_{g}, dans laquelle A et E représentent H, un métal alcalin, un métal alcalino-terreux ou néant, et où R¹ et R² ont la signification donnée ci-dessus, et 0<a<9 ; 0<b<2 ; 0<c<5 ; 0<d<2 ; 0<e<2 ; 0<f<2 ; g<0,5. Lorsque A ou E représente H, l'atome d'oxygène qui le porte n'est pas lié à la matrice phosphocalcique ou il lui est simplement associé par liaison hydrogène. Lorsque A ou E est "néant", l'atome d'oxygène qui le porte est coordiné à un autre élément de la composition, par exemple à un Ca.

La teneur en acide gem-biphosphonique d'un composé phosphocalcique modifié peut être déterminée par spectroscopie UV-visible selon la méthode décrite par Ames, B.N., notamment dans Methods in Enzymology, Colowick, S. P. and Kaplan, N. O. Eds., Academic Press, Orlando, 1966, vol. 8, pp. 115-118. Elle peut en outre être déterminée par RMN liquide ³¹P. La caractérisation du composé phosphocalcique modifié peut être effectuée essentiellement par la RMN du solide ³¹P MAS qui montre à la fois la présence du support phosphocalcique et celle du principe actif.

Un autre objet de l'invention est une composition utilisable par injection pour le traitement d'ostéoporose ou de récidives de tumeurs lytiques par inhibition de l'activité ostéoclastique. Ladite composition est une suspension du composé phosphocalcique modifié défini ci-dessus dans une solution ou un gel biocompatible présentant une viscosité permettant le transport de granules de taille comprise entre 40 µm et 500 µm. A titre d'exemple, on peut citer les hydrogels d'intérêt biologique décrits dans Chem. Rev. (2001).; 101(7) : 1869-1879, notamment les hydrogels dérivés de la cellulose ou à base d'hyaluronate de sodium.

Le choix du diamètre particulaire est guidé par la cinétique de résorption d'une part et la rhéologie à l'injection d'autre part. Les particules de diamètre inférieur à 40µm présentent une cinétique de biorésorption trop rapide et les particules de diamètre supérieur à 500 µm présentent des problèmes de rhéologie à l'injection. Il est toutefois entendu qu'une faible proportion de particules (jusqu'à 10% en volume) peut avoir un diamètre inférieur à 40µm ou supérieur à 500µm. Une composition injectable selon l'invention contient de préférence de 40 % à 75 % en masse de composé phosphocalcique modifié, de 60 % à 25 % en masse d'hydrogel, et éventuellement divers additifs. Les additifs sont choisis parmi les composés capables d'introduire divers ions d'intérêt biologique tels que par exemple . K⁺, Na⁺, Zn²⁺, Mg²⁺, CO₃²⁻, HPO₄²⁻, F⁻ ou Cl⁻.

La composition peut être préparée en mettant en suspension dans le milieu approprié le composé phosphocalcique modifié préparé dans une étape préliminaire. Il peut en outre être préparé en précipitant le composé phosphocalcique modifié in situ, à partir d'un hydrogel défini comme précédemment et préalablement chargé en ions phosphates (respectivement calcium) dans lequel sera ajoutée une solution appropriée contenant des ions calcium (respectivement phosphates) et la concentration souhaitée en acide biphosphonique.

Le mode d'association entre la matrice phosphocalcique et l'acide biphosphonique est différent suivant la matrice phosphocalcique utilisée, et cette différence se traduit par une efficacité biologique différente lors de tests in vitro sur des cultures d'ostéoclastes.

La composition selon l'invention, sous formé injectable, permet de traiter localement un problème osseux sur les principaux sites à risques identifiés (col du fémur et corps vertébraux), à l'aide d'un principe actif connu pour son utilisation par voie systémique qui présente divers inconvénients rappelés précédemment. En outre, la phase phosphocalcique, qui joue le rôle de vecteur du principe actif, exerce un effet complémentaire en ce sens qu'elle permet de maintenir l'acide gem-biphosphonique en place, et qu'elle constitue une source de calcium et de phosphate nécessaire pour la stimulation du remodelage osseux. L'hydroxyapatite (HA) décrite dans l'art antérieur comme matrice d'un implant imprégné d'un principe actif ne fait pas partie des composés phosphocalciques utilisables dans la présente invention, car elle a une solubilité relativement faible, elle possède intrinsèquement un caractère faiblement résorbable, et l'introduction d'acide gem-biphosphonique diminue le potentiel de résorbabilité des composés phosphocalciques de manière générale.

### Les exemples

La présente invention est décrite plus en détail par les exemples suivants, qui sont donnés à titre d'illustration, mais auxquels l'invention n'est pas limitée.

Les composés et réactifs suivants ont été utilisés :
■ eau ultra-pure : eau ayant une résistivité voisine de 18 MΩ cm
■ zoledronate de sodium : acide gem biphosphonique commercialisé par la société Novartis
■ tiludronate de sodium : acide gem biphosphonique commercialisé par la société Sanofi-Synthélabo
■ CDA voie NaOH : hydroxyapatite déficiente en calcium obtenue par hydrolyse de phosphate dicalcique dihydraté avec une solution aqueuse de NaOH), sous forme de granules ayant une granulométrie entre 40-80 µm)
■ CDA voie ammoniaque (hydroxyapatite déficiente en calcium obtenue par hydrolyse de phosphate dicalcique dihydraté avec de l'ammoniaque), sous forme de granules ayant une granulométrie entre 40-80 µm
■ β-TCP, sous forme de granules ayant une granulométrie entre 40-80 µm.
■ BCP (75% β-TCP / 25% HA) sous forme de granules ayant une granulométrie entre 40-80 µm
■ BCP (25% β-TCP / 75% HA) sous forme de granules ayant une granulométrie entre 40-80 µm.

### Exemple 1

### Préparation d'un composé phosphocalcique modifié

On a préparé une suspension de phosphate de calcium en introduisant 700 mg de BCP ayant une granulométrie de 40-80 µm, dans 3,5 ml d'eau ultra-pure, et on a ajouté 56 mg (0,14 mmol) de zoledronate. On a placé la suspension dans un tube maintenu à température ambiante, et on a agité par un agitateur rotatif à 16 tr/min pendant 48 h. Ensuite, on a centrifugé la suspension et on a séparé le culot du surnageant.

La phase solide a ensuite été lavée plusieurs fois avec de l'eau ultra-pure, puis filtrée et séchée à température ambiante.

Le procédé a également été mis en oeuvre à partir des composés phosphocalciques suivants : CDA voie NaOH, CDA voie ammoniaque et β-TCP.

### Caractérisation des composés phosphocalciques modifiés

La quantité de zoledronate incorporée dans chacune des matrices phosphocalciques a été déterminée par différence, en dosant la quantité de zoledronate présente dans le surnageant. Ce dosage a été effectué, sur la solution surnageante séparée du culot après centrifugation, par RMN liquide ³¹P à partir de courbes d'étalonnage préalablement établies. Il peut également être effectué par spectroscopie UV-visible selon la méthode précitée décrite par Ames.

Les résultats obtenus pour chacun des composés phosphocalciques précurseurs sont donnés dans le tableau ci-dessous. T (%) indique la teneur en zoledronate dans le produit final, exprimée en mg de principe actif pour 100 mg de composé phosphocalcique, et P (%) indique le pourcentage de zoledronate fixé sur le composé par rapport à la quantité introduite dans le milieu réactionnel :

| Précurseur | T (%) | P (%) |
|---|---|---|
| BCP (75% β-TCP / 25% HA) | 1 | 13 |
| BCP (25% β-TCP / 75% HA) | 2,7 | 33 |
| CDA voie NaOH | 5,2 | 65 |
| CDA voie ammoniaque | 6,4 | 80 |
| β-TCP | 6,4 | 80 |

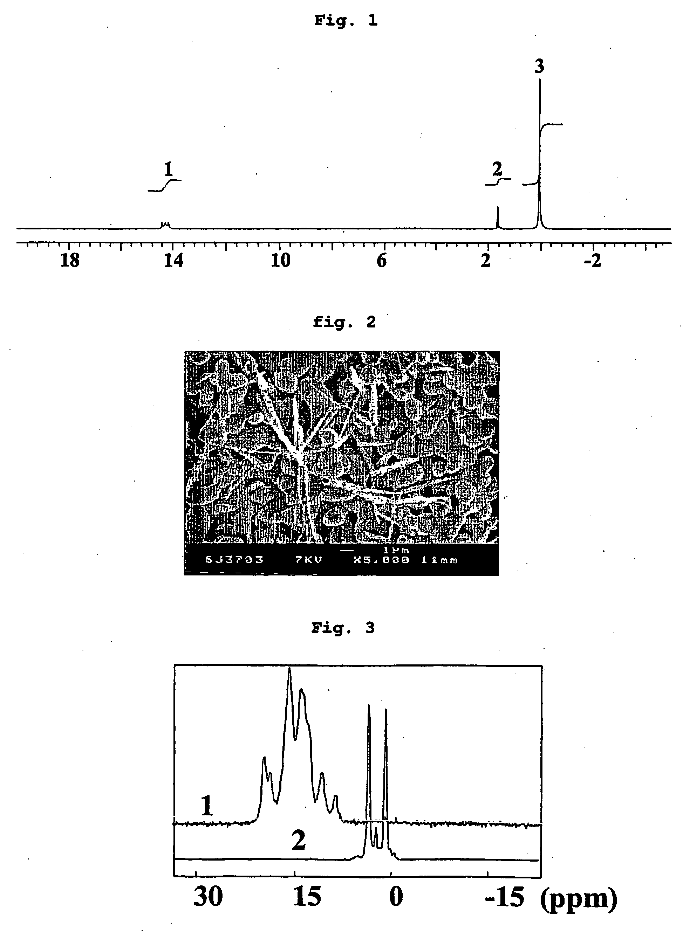

La figure 1 représente le spectre RMN liquide ³¹P du surnageant obtenu après centrifugation du milieu réactionnel correspondant au précurseur CDA (voie NaOH). L'intégration des signaux rend compte de l'abondance de chaque espèce et le déplacement chimique (caractéristique de l'espèce) est porté en abscisse. Le pic 1 représente la teneur en zoledronate, le pic 2 représente le phosphate relargué dans le milieu par le composé phosphocalcique et le pic 3 représente la référence NaH₂PO₄.

Le spectre RMN liquide ³¹P des composés obtenus à partir des autres précurseurs (sauf l'hydroxyapatite) est analogue et l'on remarque dans tous les cas un relargage de phosphate en cours de réaction.

La caractérisation des solides obtenus montre deux modes d'association différents du zoledronate suivant la nature du composé phosphocalcique de départ. La figure 2 représente une photographie par microscopie électronique à balayage (M.E.B.) effectuée sur le composé issu du β-TCP. Elle montre qu'une forme de zoledronate (probablement associée à du calcium) cristallise à la surface de la matrice phosphocalcique. Le même phénomène est observé dans le cas des BCP, qu'ils soient riches en β-TCP (75% β-TCP - 25% HA) ou pauvres en β-TCP (25% β-TCP - 275% HA).

Les données RMN du solide ³¹P MAS sont représentées sur la figure 3 pour le composé issu du β-TCP. Le spectre 1 acquis en mode CP (Polarisation Croisée) permet d'observer sélectivement le zoledronate incorporé. Les signaux fins rendent compte de sa présence sous une forme cristalline. Le spectre 2 enregistré en mode découplage proton permet d'observer sélectivement le support β-TCP non altéré.

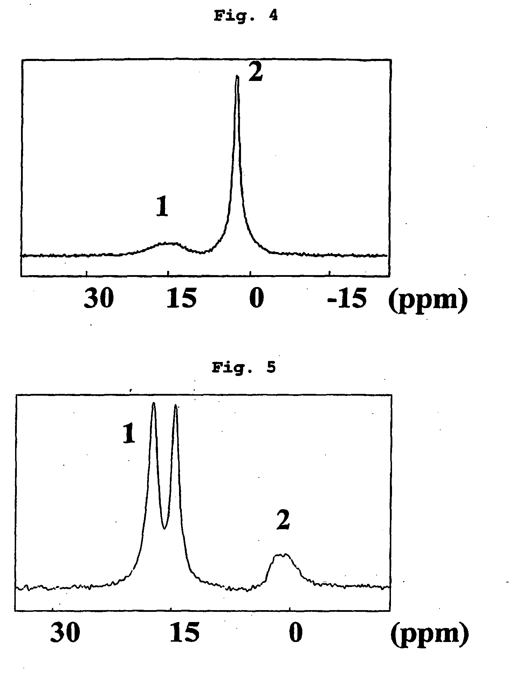

Le spectre RMN du solide ³¹P CP-MAS est représenté sur la figure 4 pour le composé issu du CDA (voie ammoniaque). Le signal du zoledronate (pic 1) est très large. Aucune phase cristalline n'est détectée à la surface du matériau, ce qui indique probablement une chimisorption du zoledronate à la surface du CDA. Le pic 2 est caractéristique du CDA.

### Exemple 2

### Préparation de composés phosphocalciques modifiés par le tiludronate et l'acide méthylène biphosphonique

On a préparé une suspension de phosphate de calcium en introduisant 700 mg de β-TCP ayant une granulométrie de 40-80 µm, dans 3,5 ml d'eau ultra pure, et on a ajouté 52,5 mg (0,14 mmol) de tiludronate. On a placé la suspension dans un tube maintenu à température ambiante, et on a agité par un agitateur rotatif à 16 tr/min pendant 48 h. ensuite, on a centrifugé la suspension et on a séparé le culot du surnageant.

La phase solide a ensuite été lavée plusieurs fois avec de l'eau ultra-pure, puis filtrée et séchée à température ambiante.

Une réactivité semblable à celle enregistrée avec le zoledronate est observée. La figure 5 montre le spectre RMN ³¹P CP-MAS du β-TCP traité par le tiludronate. Le tiludronate peut être observé sous forme d'une phase cristalline (massif 1 constitué de signaux fins) déposée sur la phase phosphocalcique (qui apparaît faiblement (massif 2) dans ces conditions d'enregistrement du spectre.

### Exemple 3

### Préparation d'une composition injectable

On a préparé une composition injectable à partir de chacun des composés modifiés obtenus dans les exemples 1 et 2, à l'exception des composés modifiés obtenus à partir d'hydroxyapatite, selon le procédé suivant.

Pour chaque composé modifié, on a préparé des granules dont 95% en volume avaient un diamètre particulaire équivalent compris entre 40 et 80 µm, et on a introduit ces granules dans une solution aqueuse contenant 3% d'hydroxypropylméthylcellulose comprenant 21% en masse de groupe méthyle et 8% en masse de groupe hydroxypropyle avec un degré de polymérisation égal à 110, de façon à obtenir une composition comprenant 49% en masse de granules.

Chacune des compositions ainsi préparées a été introduite dans un flacon de verre et stérilisée à l'autoclave à 121°C pendant 20 minutes.

### Exemple 4

### Tests in vitro de phosphates de calcium modifiés

Des cellules osseuses totales, isolées à partir d'os longs de lapins nouveaux-nés, ont été utilisées pour évaluer l'efficacité de l'association du composé phosphocalcique modifié. Les performances du BCP modifié et du CDA voie ammoniaque modifié obtenus dans l'exemple 1 ont été mesurées et comparées avec celle du précurseur phosphocalcique respectif non traité par l'acide gem-biphosphonique.

Pour chaque essai, on a chargé dans un premier puit de culture deux pastilles de dentine de cachalot (composé de référence pour la mesure de la résorption) et une pastille de composé phosphocalcique non traité, et dans un second puit de culture, deux pastilles de dentine et une pastille de composé phosphocalcique traité en surface.

L'activité de résorption des ostéoclastes dans ces conditions de culture a été évaluée (après 5 jours) par trois paramètres différents :
1- Le nombre de total de lacunes formées à la surface de la dentine de cachalot
2- La surface moyenne des lacunes
3- La surface de dentine résorbée.

Il apparaît que :
■ En présence de pastilles de BCP modifiées par 1 % de zoledronate en poids, l'activité de résorption résiduelle des cellules osseuses du modèle était indétectable. Ce phénomène serait lié à un relargage important de zoledronate ayant eu un effet cytotoxique. En effet, si l'on met du BCP ou du β-TCP modifié dans de l'eau, un pourcentage significatif du zoledronate chargé repasse rapidement en solution. Par exemple, 60 mg de β-TCP modifié mis en suspension dans 1 ml d'eau pendant 8 heures conduit au relargage d'environ 25 % du zoledronate chargé, soit une concentration molaire de 10⁻² M.
■ En présence de pastilles de CDA modifiées par 6,4 % de zoledronate en poids, l'activité de résorption des cellules était réduite d'environ 80 % par rapport au contrôle sans zoledronate. Comme dans le cas du β-TCP modifié, si l'on met en suspension 60 mg de CDA modifiée dans 1 ml d'eau pendant 8 heures, aucune trace de zoledronate n'est détectée (méthode UV-visible). Ceci implique que le zoledronate n'est potentiellement présent qu'à des concentrations inférieures à 10⁻⁴ M (limite de détection dans nos conditions d'analyse).

Ces résultats montrent que les performances du matériau résultent non pas de la seule quantité de zoledronate fixée sur la matrice phosphocalcique, mais aussi de la vitesse de relargage du zolédronate, et ils confirment un effet à distance de la matrice phosphocalcique modifiée.

### Exemple 5

Plusieurs échantillons de CDA (200 mg) modifiée par ajout de zoledronate conformément au procédé de l'exemple 1 et plusieurs échantillons de CDA non modifiée ont été incubés dans 5 ml de milieu de culture à 37 °C. Après 96 heures d'incubation, les différents surnageants ont été recueillis et utilisés purs, dilués 10, 100 et 1000 fois dans un modèle d'ostéoclastes de lapins.

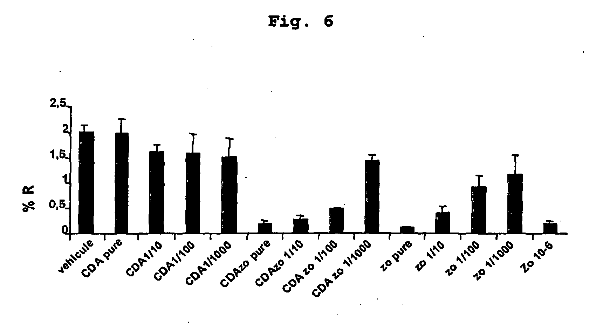

Les résultats sont représentés sur la figure 6, sur laquelle le pourcentage de résorption R est indiqué en ordonnées, les conditions étant indiquées en abscisse. Parmi les conditions :
- "vehicule" signifie le milieu de culture seul
- "CDA pure" signifie le surnageant issu de l'incubation de CDA pure dans le milieu de culture ; "CDA1/10", "CDA1/100" et "CDA1/1000" signifient respectivement la solution désignée ci-dessus et diluée à 1/10, 1/100 et 1/1000.
- "zo pure" signifie une solution de zoledronate 10⁻⁶ M dans le milieu de culture, "zo1/10", "zo1/100", "zo1/1000" signifient respectivement la solution désignée ci-dessus et diluée à 1/10, 1/100 et 1/1000.
- "CDAzo pure" signifie le surnageant issu de l'incubation de CDA chargée en zoledronate dans le milieu de culture "CDAzol/10", "CDAzol/100" et "CDAzol/1000" signifient respectivement la solution désignée ci-dessus et diluée à 1/10, 1/100 et 1/1000.

Ces résultats montrent que :
- le zoledronate libéré par la phase phosphocalcique (CDAzo) conserve son activité inhibitrice de la résorption ostéoclastique avec un effet dose marqué,
- la CDA seule ne semble pas influencer la résorption ostéoclastique quelle que soit la dilution du surnageant, 1
- le zoledronate en solution (zo) conserve son activité biologique et inhibe selon une relation dose/effet la résorption.

La comparaison des profils d'inhibition de la résorption ostéoclastique provoquée par l'association CDA/Zo et par le zoledronate seul (solution 10⁻⁶ M utilisée pure, diluée 10, 100 et 1000 fois) permet de suggérer que le matériau libère une quantité de zoledronate correspondant à une concentration de l'ordre de 10⁻⁶ M.

## Revendications

1. Procédé de préparation d'un composé phosphocalcique modifié, **caractérisé en ce qu'**il consiste à ajouter un acide gem-biphosphonique ou l'un de ses sels de métal alcalin ou alcalino-terreux à une suspension d'un composé phosphocalcique précurseur dans l'eau ultra-pure, à agiter le milieu réactionnel à température ambiante, puis en récupérer par centrifugation le composé formé.

2. Procédé selon la revendication 1, **caractérisé en ce que** tes acides ou les sels utilisés comme composés gem-biphosphoniques répondent à la formule (OY)(OX)P(O)-CR¹CR²-P(O)(OX)(OY) dans laquelle X ou Y représentent indépendamment l'un de l'autre H ou un cation de métal alcalin ou alcalino-terreux, R¹ représente H, OH ou un halogène, et R² représente :
• un hydrogène ou un halogène,
• un radical alkyle,
• un radical aminoalkyle dans lequel le groupe amino porte éventuellement un substituant alkyle,
• un radical alkylamino,
• un radical alkyle portant un substituant aromatique comprenant au moins un atome N,
• un radical alkyle portant un groupe thioéther aromatique.

3. Procédé selon la revendication 2, **caractérisé en ce que** R¹ et/ou R² représentent Cl.

4. Procédé selon la revendication 2, **caractérisé en ce que** R² est un radical ayant de 1 à 6 atomes de carbone.

5. Procédé selon la revendication 2, **caractérisé en ce que** R² est un radical aminoalkyle NH₂(CH)ₙ- dans lequel n est inférieur à 6.

6. Procédé selon la revendication 2, **caractérisé en ce que** R² est un radical alkylaminoalkyle R'R"N(CH₂)ₘ- dans lequel R' et R" représentent indépendamment l'un de l'autre H ou un radical alkyle ayant au plus 5 atomes de carbone, et m est inférieur à 6.

7. Procédé selon la revendication 2, **caractérisé en ce que** R² est un radical alkylamino R^{c}NH- dans lequel R^{c} est un cycloalkyle ayant de 3 à 7 atomes de carbone.

8. Procédé selon la revendication 2, **caractérisé en ce que** R² est un radical alkyle ayant au plus 3 atomes de carbone et portant un groupe pyridinyle ou imidazolyle,

9. Procédé selon la revendication 2, **caractérisé en ce que** R² est un radical alkyle ayant au plus 3 atomes de carbone et portant un groupe phénylthio dans lequel le groupe phényle porte éventuellement un substituant halogène.

10. Procédé selon la revendication 2, **caractérisé en ce que** R¹ est OH, R² est -CH₂-imidazole, A et E représentent H.

11. Procédé selon la revendication 1, **caractérisé en ce que** le composé formé est purifié par lavage à l'eau ultra-pure, suivi d'une filtration et d'un séchage à l'air à température ambiante.

12. Procédé selon la revendication 1, **caractérisé en ce que** le composé phosphocalcique est choisi parmi le BCP, la CDA qui est une hydroxyapatite déficiente en calcium et le β-TCP.

13. Procédé selon la revendication 1, **caractérisé en ce que** l'agitation à température ambiante est maintenue pendant une durée entre 1 h et 72 h.

14. Composé phosphocalcique modifié susceptible d'être obtenu par le procédé selon l'une des revendications 1 à 13.

15. Composé phosphocalcique modifié selon la revendication 14, dans lequel le composé phosphocalcique est choisi parmi le BCP, la CDA qui est une hydroxyapatite déficiente en calcium et le β-TCP.

16. Composition utilisable par injection pour le traitement d'ostéoporose ou de récidives de tumeurs lytiques par inhibition de l'activité ostéoclastique, **caractérisée en ce qu'**elle est constituée par une suspension de 40 à 75% en masse du composé phosphocalcique modifié selon l'une des revendications 14 ou 15, dans 60 à 25% en masse d'un hydrogel.

17. Composition selon la revendication 16, **caractérisée en ce que** le gel biocompatible est un hydrogel d'intérêt biologique.

18. Composition selon la revendication 17, **caractérisée en ce que** le gel est un hydrogel dérivé de la cellulose ou un gel à base d'hyaluronate de sodium.

## Claims

1. Method for preparation of a modified phosphocalcic compound, **characterised in that** it comprises addition of a gem-biphosphonic acid or one of its alkali-earth or alkali metal salts to a suspension of a precursor phosphocalcic compound in ultra-pure water, agitation of the reaction medium at ambient temperature, then recovery of the formed compound by centrifuging.

2. Method according to Claim 1, **characterised in that** the acids or salts used as gem-biphosphonic compounds correspond to the formula (OY)(OX)P(O)-CR¹CR²-P(O)(OX)(OY) in which X and Y represent, independently of each other, H or a cation of alkali-earth or alkali metal, R¹ represents H, OH or a halogen, and R² represents:
• a hydrogen or a halogen
• an alkyl radical
• an amino-alkyl radical in which the amino group may carry an alkyl substitute
• an alkylamino radical
• an alkyl radical carrying an aromatic substitute comprising at least one N atom,
• an alkyl radical carrying an aromatic thio-ether group.

3. Method according to Claim 2, **characterised in that** R¹ and/or R² represent CI.

4. Method according to Claim 2, **characterised in that** R² is a radical with 1 to 6 carbon atoms.

5. Method according to Claim 2, **characterised in that** R² is an amino-alkyl radical NH₂(CH)ₙ- in which n is less than 6.

6. Method according to Claim 2, **characterised in that** R² is an alkylaminoalkyl radical R'R"N(CH₂)ₘ- in which R' and R" represent, independently of each other, H or an alkyl radical with maximum 5 carbon atoms and m is less than 6.

7. Method according to Claim 2, **characterised in that** R² is an alkylamino radical R^{c}NH- in which R^{c} is a cyclo-alkyl with 3 to 7 carbon atoms.

8. Method according to Claim 2, **characterised in that** R² is an alkyl radical with maximum 3 carbon atoms and carrying a pyridinyl or imidazolyl group.

9. Method according to Claim 2, **characterised in that** R² is an alkyl radical with maximum 3 carbon atoms and carrying a phenylthio group in which the phenyl group may carry a halogen substitute.

10. Method according to Claim 2, **characterised in that** R¹ is OH, R² is -CH₂-imidazole, A and E represent H.

11. Method according to Claim 1, **characterised in that** the compound formed is purified by washing in ultra-pure water, followed by filtration and air drying at ambient temperature.

12. Method according to Claim 1, **characterised in that** the phosphocalcic compound is selected from BCP, CDA which is a hydroxyapatite deficient in calcium and β-TCP.

13. Method according to Claim 1, **characterised in that** the agitation at ambient temperature is maintained for a duration between 1 h and 72 h.

14. Modified phosphocalcic compound able to be obtained by the method according to any one of Claims 1 to 13.

15. Modified phosphocalcic compound according to Claim 14, in which the phosphocalcic compound is selected from BCP, CDA which is a hydroxyapatite deficient in calcium and β-TCP.

16. Compound which can be used by injection for treatment of osteoporosis or recurrence of lytic tumours by inhibition of osteoclastic activity, **characterised in that** it comprises a suspension of 40 to 75% mass of modified phosphocalcic compound according to either of Claims 14 and 15, in 60 to 25% mass of a hydrogel.

17. Compound according to Claim 16, **characterised in that** the biocompatible gel is a hydrogel of biological interest.

18. Compound according to Claim 17, **characterised in that** the gel is a hydrogel derived from cellulose or a gel based on sodium hyaluronate.

## Patentansprüche

1. Verfahren zur Herstellung einer modifizierten Calciumphosphatverbindung, **dadurch gekennzeichnet, dass** es aus dem Hinzufügen einer gem-Diphosphonsäure oder eines ihrer Alkalimetall- oder Erdalkalimetallsalze zu einer Suspension einer Calciumphosphat-Vorläuferverbindung in ultrareinem Wasser, dem Rühren des Reaktionsmediums bei Raumtemperatur und darauf Gewinnen der gebildeten Verbindung durch Zentrifugieren besteht.

2. Verfahren gemäß Anspruch 1, **dadurch gekennzeichnet, dass** die als *gem-*Diphosphonverbindungen verwendeten Säuren oder Salze der Formel (OY)(OX)P(O)-CR¹CR²-P(O)(OX)(OY) entsprechen, worin X oder Y unabhängig voneinander H oder ein Alkalimetall- oder Erdalkalimetallkation darstellen, R¹ H, OH oder ein Halogen darstellt und R² Folgendes darstellt:
• Wasserstoff oder ein Halogen,
• einen Alkylrest,
• einen Aminoalkylrest, bei dem die Aminogruppe gegebenenfalls einen Alkylsubstituenten trägt,
• einen Alkylaminorest,
• einen Alkylrest, der einen wenigstens ein N-Atom enthaltenden aromatischen Substituenten trägt,
• einen Alkylrest, der eine aromatische Thioethergruppe trägt.

3. Verfahren gemäß Anspruch 2, **dadurch gekennzeichnet, dass** R¹ und/oder R² CI darstellen.

4. Verfahren gemäß Anspruch 2, **dadurch gekennzeichnet, dass** R² ein Rest mit 1 bis 6 Kohlenstoffatomen ist.

5. Verfahren gemäß Anspruch 2, **dadurch gekennzeichnet, dass** R² ein Aminoalkylrest NH₂(CH)ₙ- ist, worin n kleiner als 6 ist.

6. Verfahren gemäß Anspruch 2, **dadurch gekennzeichnet, dass** R² ein Alkylaminoalkylrest R'R"N(CH₂)ₘ- ist, worin R' und R" unabhängig voneinander H oder einen Alkylrest mit höchstens 5 Kohlenstoffatomen darstellen und m kleiner als 6 ist.

7. Verfahren gemäß Anspruch 2, **dadurch gekennzeichnet, dass** R² ein Alkylaminorest R^{c}NH- ist, worin R^{c} Cycloalkyl mit 3 bis 7 Kohlenstoffatomen ist.

8. Verfahren gemäß Anspruch 2, **dadurch gekennzeichnet, dass** R² ein Alkylrest mit höchstens 3 Kohlenstoffatomen ist und eine Pyridinyl- oder Imidazolylgruppe trägt.

9. Verfahren gemäß Anspruch 2, **dadurch gekennzeichnet, dass** R² ein Alkylrest mit höchstens 3 Kohlenstoffatomen ist und eine Phenylthiogruppe trägt, wobei die Phenylgruppe gegebenenfalls einen Halogensubstituenten trägt.

10. Verfahren gemäß Anspruch 2, **dadurch gekennzeichnet, dass** R¹ OH ist, R² -CH₂-Imidazol ist und A und E H darstellen.

11. Verfahren gemäß Anspruch 1, **dadurch gekennzeichnet, dass** die gebildete Verbindung durch Waschen mit ultrareinem Wasser, gefolgt von der Filtration und dem Trocknen an der Luft bei Raumtemperatur gereinigt wird.

12. Verfahren gemäß Anspruch 1, **dadurch gekennzeichnet, dass** die Calciumphosphatverbindung aus BCP, CDA, das ein calciumarmer Hydroxyapatit ist, und *β-*TCP ausgewählt ist.

13. Verfahren gemäß Anspruch 1, **dadurch gekennzeichnet, dass** das Rühren bei Raumtemperatur über eine Dauer zwischen 1 h und 72 h aufrechterhalten wird.

14. Modifizierte Calciumphosphatverbindung, die durch das Verfahren gemäß einem der Ansprüche 1 bis 13 erhalten werden kann.

15. Modifizierte Calciumphosphatverbindung gemäß Anspruch 14, wobei die Calciumphosphatverbindung aus BCP, CDA, das ein calciumarmer Hydroxyapatit ist, und TCP ausgewählt ist.

16. Durch Injektion zur Behandlung von Osteoporose oder Rezidiven lytischer Tumoren durch Hemmung der Osteoklastenaktivität verwendbare Zusammensetzung, **dadurch gekennzeichnet, dass** sie sich aus einer Suspension von 40 bis 75 Gew.-% modifizierter Calciumphosphatverbindung gemäß einem der Ansprüche 14 oder 15 in 60 bis 25 Gew.-% eines Hydrogels zusammensetzt.

17. Zusammensetzung gemäß Anspruch 16, **dadurch gekennzeichnet, dass** das bioverträgliche Gel ein Hydrogel von biologischem Interesse ist.

18. Zusammensetzung gemäß Anspruch 17, **dadurch gekennzeichnet, dass** das Gel ein von Cellulose abgeleitetes Hydrogel oder ein Gel auf der Grundlage von Natriumhyaluronat ist.
